# FASCICULE DE BREVET EUROPEEN

(11) **EP 2 079 438 B1**
(45) Date de publication et mention de la délivrance du brevet: **23.12.2015**
(21) Numéro de dépôt: 07858398.6
(22) Date de dépôt: 05.10.2007
(51) Int. Cl.: A61K 8/97, A61Q 19/00, A61K 8/49

(54) **UTILISATION D'UNE COMPOSITION COSMETIQUE POUR LE SOIN DES PEAUX GRASSES**
VERWENDUNG EINER KOSMETISCHEN ZUSAMMENSETZUNG ZUR PFLEGE VON FETTIGER HAUT
USE OF A COSMETIC COMPOSITION FOR THE CARE OF FATTY SKIN

(30) Priorité: 06.10.2006 FR 0608785
(43) Date de publication de la demande: 22.07.2009
(73) Titulaire: Laboratoires Clarins, 92200 Neuilly sur Seine (FR)
(72) Inventeur: COURTIN, Olivier, 92100 Boulogne Billancourt (FR)
(74) Mandataire: Corizzi, Valérie
(86) Numéro de dépôt international: PCT/FR2007/001628
(87) Numéro de publication internationale: WO 2008/043900

(56) Documents cités:
- WO-A-2004/089392
- WO-A-2005/102266
- DE-A1- 10 118 382
- DE-A1- 19 807 774
- JP-A- 1 047 708
- JP-A- 3 005 423
- JP-A- 2000 319 154
- JP-A- 2001 213 775
- US-B1- 6 423 747

## Description

La présente invention concerne l'utilisation d'une composition cosmétique pour prévenir et traiter les problèmes des peaux grasses.

On peut distinguer selon l'hydratation de la couche cornée et sa production de sébum, trois types principaux de peau : les peaux sèches, les peaux grasses et les peaux mixtes.

La connaissance des différents types de peau permet d'adapter un traitement cosmétique approprié pour chaque cas.

La peau est constituée, entre autre, de glandes sébacées situées au niveau du derme et presque toujours associées à un poil. Toutes les parties du corps en sont pourvues, à l'exception de la plante des pieds et de la paume des mains. Certaines zones comme le visage, le cuir chevelu, le torse en comportent un plus grand nombre (environ cinq millions). Ces glandes sébacées sécrètent le sébum qui s'écoule le long du canal pilaire jusqu'à la surface de la peau où, au contact de la sueur, il forme le film hydrolipidique qui hydrate la couche cornée et la protège.

La glande sébacée est une glande acineuse en grappe. Elle est constituée de nombreuses couches cellulaires dans lesquelles on trouve deux types de cellules. D'une part des cellules indifférenciées (couche germinative), situées vers la périphérie de la glande, qui se divisent activement. Ces cellules migrent en 2 semaines environ vers le centre pour donner des cellules différenciées. D'autre part des cellules différenciées centrales (sébocytes) qui contiennent l'équipement enzymatique nécessaire à la synthèse des lipides. Ces cellules ne se divisent plus. En 8 jours elles se transforment en cellules matures plus grosses, remplies de sébum ; les lipides y sont synthétisés et stockés pour constituer finalement de grosses vacuoles.

Dans l'espèce humaine, chaque follicule se développe selon son propre cycle, c'est à dire qu'une glande peut s'atrophier pendant qu'une autre s'hypertrophie. Le renouvellement des sébocytes est d'environ 3 semaines.

Le volume glandulaire dépend de l'activité proliférative du compartiment germinatif, du temps nécessaire à la différenciation du sébocyte et de la qualité du sébum synthétisé par chaque sébocyte.

Le sébum est produit en quantité plus ou moins importante, en fonction de la taille et du nombre des glandes sébacées. Une sécrétion trop abondante entraîne une modification de la peau : elle devient grasse avec un aspect luisant, son grain est épais et ses pores dilatés avec parfois des points noirs.

Outre le caractère peu esthétique d'une peau brillante, il convient de remarquer que les peaux grasses présentent aussi une tendance à être facilement irritables et une très mauvaise tenue du maquillage. Il existe donc un besoin pour une composition cosmétique capable de limiter la production de sébum afin de conserver à la surface cutanée ses caractéristiques de 'peau normale'. On a proposé dans l'art antérieur plusieurs compositions cosmétiques susceptibles d'offrir une solution aux problèmes des peaux grasses. Ces compositions contiennent souvent des poudres qui permettent d'absorber le sébum et donc de matifier la peau par un effet mécanique, ou des agents kératolitiques favorisant l'expulsion des bouchons cornés. Il existe aussi des actifs astringents permettant de lutter contre la dilatation des follicules sébacés, ou des actifs exfoliants permettant de diminuer l'épaisseur de la peau.

Toutefois, l'efficacité de ces compositions est relative, car la plupart ne limitent pas la production de sébum mais ne font qu'absorber le sébum par un effet de pompage. En outre, la plupart de ces compositions n'ont qu'un effet curatif et ne permettent pas de prévenir à plus long terme le phénomène. Enfin, le soufre actif efficace sur la régularisation de la production de sébum, n'est généralement pas utilisable dans une composition cosmétique du fait de sa mauvaise odeur.

Il subsiste donc le besoin d'une composition cosmétique qui permet de prévenir et traiter les problèmes des peaux grasses et qui n'agisse pas sur les symptômes des peaux grasses mais bien sur leurs causes.

La demanderesse a mis en évidence l'activité de la chrysine sur la production de sébum. En effet la chrysine est capable de réguler la prolifération des sébocytes humains, de retarder leur différenciation en cellules matures productrices de sébum. Les cellules produisant du sébum étant moins nombreuses, la production de sébum est amoindrie, et par conséquent la quantité de sébum déversée par les glandes sébacées à la surface cutanée est diminuée. La peau ne présente alors plus les caractéristiques inesthétiques d'une peau grasse. La chrysine possède une action à la fois curative et préventive puisqu'elle permet de moduler à long terme la production de sébum.

La présente invention concerne donc l'utilisation de la chrysine dans une composition cosmétique ou pour la préparation d'une composition dermatologique destinée au soin des peaux grasses.

La présente invention concerne aussi l'utilisation de la chrysine dans une composition cosmétique ou pour la préparation d'une composition dermatologique régulatrice des peaux grasses, et en particulier l'utilisation de la chrysine dans une composition cosmétique ou pour la préparation d'une composition dermatologique comme agent régulateur des peaux grasses.

La présente invention concerne également l'utilisation de la chrysine dans une composition cosmétique ou pour la préparation d'une composition dermatologique régulatrice de la production de sébum et en particulier l'utilisation de la chrysine dans une composition cosmétique ou pour la préparation d'une composition dermatologique comme agent régulateur de la production de sébum.

La présente invention concerne également l'utilisation de la chrysine dans une composition cosmétique ou pour la préparation d'une composition dermatologique régulatrice de la prolifération des sébocytes et en particulier l'utilisation de la chrysine dans une composition cosmétique ou pour la préparation d'une composition dermatologique comme agent régulateur de la prolifération des sébocytes.

La présente invention concerne également l'utilisation de la chrysine dans une composition cosmétique ou pour la préparation d'une composition dermatologique purifiante et/ou matifiante et en particulier l'utilisation de la chrysine dans une composition cosmétique ou pour la préparation d'une composition dermatologique comme agent purifiant et/ou matifiant. La présente invention concerne enfin l'utilisation de la chrysine dans une composition cosmétique ou pour la préparation d'une composition dermatologique protectrice anti-brillance, et en particulier l'utilisation de la chrysine dans une composition cosmétique ou pour la préparation d'une composition dermatologique comme agent protecteur anti brillance.

La chrysine est déjà utilisée en cosmétique comme agent antiviral associé à un filtre solaire dans la demande de brevet EP 0980684. L'utilisation de la chrysine comme agent anti radicalaire est également décrite dans le brevet FR 2687572. De façon surprenante la demanderesse a mis en évidence l'action régulatrice de la chrysine sur la différenciation et la prolifération des sébocytes humains.

La chrysine aussi dénommée 5,7-dihydroxyflavone est une molécule de poids moléculaire de 254.23 g/mol.

De façon avantageuse, la chrysine utilisable dans le cadre de la présente invention est obtenue par synthèse chimique et couramment disponible. Il s'agit d'une poudre de couleur jaune clair sans odeur, de teneur maximale en eau de 2% et de poids moléculaire de 254,23 g/mol. La chrysine étant présente dans de nombreuses plantes on peut aussi utiliser une chrysine d'origine naturelle.

On peut se procurer de la chrysine utilisable selon l'invention par exemple auprès de la société DKSH France, commercialisée sous la dénomination 'chrysine'.

Selon l'invention, la chrysine peut être utilisée avec un ou plusieurs autres actifs ayant des propriétés anti peaux grasses. En particulier, la chrysine peut être associée dans une composition cosmétique ou dermatologique à un extrait de Lamier blanc, aussi appelé *Lamium album.* En effet, le lamier blanc présente une teneur en soufre supérieure à 200 ppm. Comme le soufre est capable de régulariser la production de sébum, l'utilisation d'un extrait de Lamier blanc, notamment un extrait glycolique, permet de disposer de soufre dans la composition cosmétique de l'invention sans présenter d'inconvénient au niveau de l'odeur désagréable. Il peut aussi s'agir d'une fraction d'un extrait de Lamier blanc riche en soufre dès lors bien entendu que cette fraction ne présente pas l'odeur désagréable.

De même, la chrysine peut être associée dans une composition cosmétique ou dermatologique à un extrait *d'hamamélis virginiana* connu pour ses propriétés astringentes, et/ou à du chlorhydrate de piridoxine ou vitamine B6, qui réduit le flux de sébum et normalise le pH des peaux grasses, et/ou à des dérivés de zinc qui possèdent à la fois une activité astringente et une efficacité fongicide et bactéricide reconnue. On entend par dérivés de zinc les sels de zinc et en particulier le sulfate de zinc et le gluconate de zinc.

La composition selon l'invention contient de l'ordre de 0.001% à 1% en poids, et de préférence 0.001% à 0.01% en poids de chrysine.

La composition cosmétique de la présente invention à application topique peut constituer notamment une composition de protection, de nettoyage, de traitement ou de soin cosmétique ou dermatologique pour le visage, pour le cou, ou pour le corps, comme, par exemple, crèmes de jour, crèmes de nuit, lotions pour le visage, masques, gels nettoyants moussants, laits corporels, une composition capillaire (par exemple une lotion pour le cuir chevelu), ou une composition de maquillage (par exemple fond de teint, crème teintée).

La composition cosmétique selon la présente invention peut contenir un ou plusieurs autres composants connus de l'homme du métier, comme des agents de formulation ou additifs d'usage connu et classique dans les compositions cosmétiques. A titre d'exemple et de façon non limitative, de tels agents de formulation et additifs peuvent être des gélifiants hydrophiles ou lipophiles, des adoucissants, des colorants, des agents solubilisants, des agents de texture, des parfums, des charges, des actifs filmogènes, des conservateurs, des tensio-actifs, des émulsionnants, des huiles, des glycols, des vitamines, des filtres solaires,.... Grâce à ses connaissances en matière de cosmétiques, l'homme du métier saura quels agents de formulation ajouter à la composition cosmétique selon l'invention et en quelles quantités en fonction des propriétés recherchées.

De plus, la composition cosmétique selon la présente invention peut se présenter sous toute forme connue de l'homme du métier dans le domaine de la cosmétique sans aucune autre restriction galénique particulière que celle pour l'application sur la peau. Ainsi, la composition cosmétique selon l'invention peut avoir la forme d'une solution ou suspension aqueuse, alcoolique ou d'une suspension huileuse ou d'une solution ou d'une dispersion de type lotion ou sérum, d'une émulsion de consistance liquide ou semi-liquide de type lait, obtenues par dispersion d'une phase grasse dans une phase aqueuse (émulsion Huile dans Eau : H/E) ou inversement (Eau dans Huile : E/H), ou d'une émulsion du type crème H/E ou E/H ou d'un gel, d'une lotion, d'un masque. On peut également envisager les formulations cosmétiques selon l'invention sous la forme d'une mousse ou encore sous forme de compositions pour aérosol comprenant également un agent propulseur sous pression.

La présente invention concerne également un procédé de traitement cosmétique pour prévenir ou traiter les problèmes des peaux grasses, comprenant l'application sur la peau d'une composition cosmétique comportant de la chrysine.

La présente invention concerne aussi l'utilisation de la chrysine pour la préparation d'une composition dermatologique destinée à prévenir ou traiter les problèmes des peaux grasses.

Les exemples ci-après concernent, d'une part l'évaluation de l'effet de la chrysine sur la prolifération des sébocytes humains en culture et d'autre part des compositions objets de la présente invention.

Les exemples font références aux figures suivantes dans lesquelles :

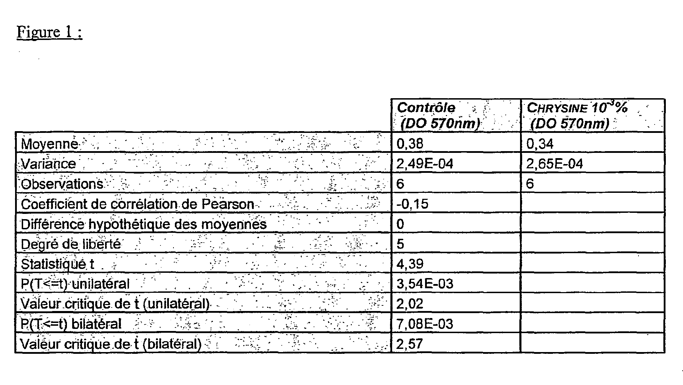

- la figure 1 représente le test d'égalité des espérances : observations pairées pour une concentration de chrysine de 10⁻³%.

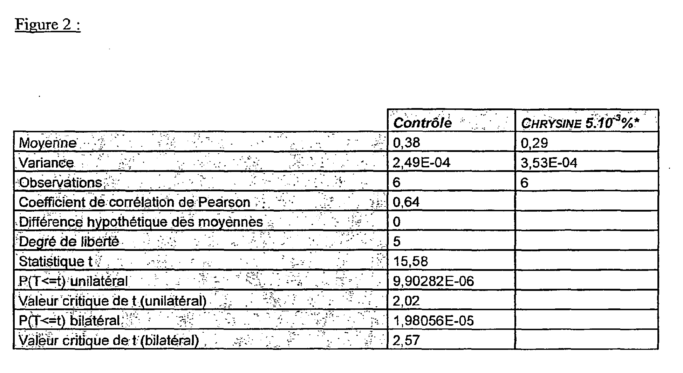

- la figure 2 représente le test d'égalité des espérances : observations pairées pour une concentration de chrysine de 5.10⁻³%.

### I.EVALUATION DE L'INHIBITION DE LA CHRYSINE SUR LA PROLIFERATION DES SEBOCYTES HUMAINS EN CULTURE.

### A.MATERIEL ET METHODE

### 1.Culture de sébocytes

Une lignée de sébocytes humains (Hs 917.T, lignée ATCC n°CRL-7669) a été mise en culture dans un milieu de Eagle modifié selon Dulbecco avec 4,5 g/l de D-Glucose et 10% de sérum de veau foetal. Ces cellules adhérentes ont une morphologie polygonale et de grands noyaux.

Elles prolifèrent en augmentant de taille et se différencient progressivement.

### 2.Evaluation de la prolifération des sébocytes

La prolifération a été évaluée par un test colorimétrique validé, le test M.T.T. (diméthyl thiazol diphényl tétrazolium) (selon Mosmann J Immunol Meth 1983 65 : 55-63). Métabolisé par les mitochondries pour donner des cristaux de formazan bleus dont la quantité dépend directement de l'activité de l'enzyme mitochondriale succinate déshydrogénase et du nombre de cellules vivantes.

On mesure, de ce fait, par colorimétrie (570 nm) la prolifération.

### 3.Mise en contact des sébocytes avec la chrysine

Dans un premier temps, nous avons vérifié la viabilité (test d'exclusion au MTT) des sébocytes en présence de la chrysine à différentes concentrations, en comparaison avec leur milieu de culture classique. Les résultats montrent que la chrysine appliquée aux concentrations 10⁻³% et 5.10⁻³% sur des sébocytes n'induisaient aucune cytotoxicité sur ces cellules.

La prolifération a donc été évaluée selon les conditions suivantes :
- sébocytes en milieu de référence :
- sébocytes cultivés en présence du milieu de référence additionné de la chrysine aux 2 concentrations 10⁻³% et 5.10⁻³%.

Pour chaque condition réalisée en sextuplé, la prolifération a été analysée après 72 heures de culture.

### B.RESULTATS

On constate un effet inhibiteur de la chrysine sur la prolifération des sébocytes humains en culture : différence de -10.5% pour une concentration de chrysine de 10⁻³% et de -18.4% pour une concentration de 5.10⁻³%.

La chrysine en inhibant la prolifération des sébocytes réduit la production de sébum. La quantité de sébum déversé à la surface cutanée étant moins importante, la peau retrouve les caractéristiques d'une `peau normale'.

### II.EXEMPLES

Dans les exemples suivants la chrysine est au préalable solubilisée dans une solution EAU 2% et NaOH 0.004 % (nommée SOL ci après) avant d'être ajoutée à la composition cosmétique.

### A.CREME POUR PEAUX GRASSES

| | % |
|---|---|
| GOMME XANTHANE | 0,10 |
| GLYCERINE | 5,00 |
| CETEARYL GLUCOSIDE | 3,00 |
| MONOSTEARATE DE GLYCEROL AE | 2,00 |
| TRYGLYCERIDE C₈C₁₀ | 10,00 |
| HUILE DE SILICONE | 3,00 |
| GLUCONATE DE ZINC | 0,02 |
| CHLORHYDRATE DE PIRIDOXINE | 0,10 |
| CHRYSINE solubilisée dans SOL | 0,0016 |
| CONSERVATEURS | 1,00 |
| PARFUM | 0,30 |
| EAU DEMINERALISEE | Q.S.P 100 |

### B.GEL POUR PEAUX GRASSES

| | % |
|---|---|
| GLYCERINE | 3,00 |
| GOMME XANTHANE | 0,20 |
| ETHANOL | 5,00 |
| GLUCONATE DE ZINC | 0,02 |
| CHLORHYDRATE DE PIRIDOXINE | 0,10 |
| CHRYSINE solubilisée dans SOL | 0,0016 |
| CONSERVATEURS | 0,50 |
| SOLUBILISANT | 0,50 |
| PARFUM | 0,20 |
| EAU DEMINERALISEE | Q.S.P 100 |

### C.LOTION POUR PEAUX GRASSES

| | % |
|---|---|
| GLYCOL .......................... | 2,00 |
| CHLORURE DE SODIUM .............. | 1,00 |

| | |
|---|---|
| ETHANOL | 5,00 |
| GLUCONATE DE ZINC | 0,02 |
| CHLORHYDRATE DE PIRIDOXINE | 0,10 |
| CHRYSINE solubilisée dans SOL | 0,0016 |
| CONSERVATEURS | 0,50 |
| SOLUBILISANT | 0,30 |
| PARFUM | 0,10 |
| EAU DEMINERALISEE | Q.S.P 100 |

## Revendications

1. Utilisation d'une composition cosmétique comprenant de la chrysine comme agent régulateur des peaux grasses.

2. Utilisation d'une composition cosmétique comprenant de la chrysine comme agent purifiant.

3. Utilisation d'une composition cosmétique comprenant de la chrysine comme agent matifiant.

4. Utilisation d'une composition cosmétique comprenant de la chrysine comme agent anti brillance.

5. Utilisation d'une composition cosmétique comprenant de la chrysine comme agent régulateur de la production de sébum.

6. Utilisation d'une composition cosmétique comprenant de la chrysine comme agent régulateur de la prolifération des sébocytes.

7. Utilisation selon l'une quelconque des revendications précédentes, **caractérisée en ce que** la composition cosmétique contient de l'ordre de 0.001% à 1% en poids, et de préférence 0.001% à 0.01% en poids de chrysine.

8. Utilisation selon l'une quelconque des revendications précédentes, **caractérisée en ce que** la composition cosmétique contient en outre au moins un actif choisi parmi un extrait de *Lamium album,* un extrait *d'hamamélis virginiana,* du chlorhydrate de piridoxine et des dérivés de zinc.

9. Procédé de traitement cosmétique pour prévenir ou traiter les problèmes des peaux grasses, comprenant l'application sur la peau d'une composition cosmétique comportant de la chrysine.

## Patentansprüche

1. Verwendung einer Chrysin umfassenden kosmetischen Zusammensetzung als Regulierungsmittel für fettige Haut.

2. Verwendung einer Chrysin umfassenden kosmetischen Zusammensetzung als Reinigungsmittel.

3. Verwendung einer Chrysin umfassenden kosmetischen Zusammensetzung als Mattierungsmittel.

4. Verwendung einer Chrysin umfassenden kosmetischen Zusammensetzung als Antiglanzmittel.

5. Verwendung einer Chrysin umfassenden kosmetischen Zusammensetzung als Regulierungsmittel für die Talgproduktion.

6. Verwendung einer Chrysin umfassenden kosmetischen Zusammensetzung als Regulierungsmittel für die Proliferation von Sebozyten.

7. Verwendung nach einem der vorhergehenden Ansprüche, **dadurch gekennzeichnet, dass** die kosmetische Zusammensetzung Chrysin im Bereich von 0,001 Gew.-% bis 1 Gew.-% und vorzugsweise von 0,001 Gew.-% bis 0,01 Gew.-% umfasst.

8. Verwendung nach einem der vorhergehenden Ansprüche, **dadurch gekennzeichnet, dass** die kosmetische Zusammensetzung weiterhin mindestens einen Wirkstoff umfasst, der ausgewählt ist aus einem Extrakt aus *Lamium album,* einem Extrakt aus *Hamamelis virginiana,* Pyridoxinhydrochlorid und Zink-Derivaten.

9. Verfahren zur kosmetischen Behandlung zur Vorbeugung oder Behandlung von Problemen der fettigen Haut, umfassend die Anwendung einer Chrysin umfassenden kosmetischen Zusammensetzung auf der Haut.

## Claims

1. Use of a cosmetic composition comprising chrysin as an agent for controlling greasy skin.

2. Use of a cosmetic composition comprising chrysin as a purifying agent.

3. Use of a cosmetic composition comprising chrysin as a mattifying agent.

4. Use of a cosmetic composition comprising chrysin as an anti-shine agent.

5. Use of a cosmetic composition comprising chrysin as an agent for controlling the production of sebum.

6. Use of a cosmetic composition comprising chrysin as an agent for controlling the proliferation of sebocytes.

7. Use according to any one of the preceding claims, **characterised in that** the cosmetic composition contains in the order of from 0.001% to 1% by weight, and preferably from 0.001% to 0.01% by weight, of chrysin.

8. Use according to any one of the preceding claims, **characterised in that** the cosmetic composition further contains at least one active ingredient which is selected from an extract of *Lamium album,* an extract of *Hamamelis virginiana,* piridoxine hydrochloride and derivatives of zinc.

9. Cosmetic treatment process for preventing or treating problems of greasy skin, comprising the application of a cosmetic composition comprising chrysin to the skin.
